Europäisches Patentamt

European Patent Office

Office européen des brevets

(11) Publication number: **0 378 710**
**A1**

## (12) EUROPEAN PATENT APPLICATION

(21) Application number: **88201204.0**

(22) Date of filing: **10.06.88**

(51) Int. Cl.5: **C07H 15/04**

(43) Date of publication of application:
**25.07.90 Bulletin 90/30**

(84) Designated Contracting States:
**AT BE CH DE ES FR GB GR IT LI NL SE**

(71) Applicant: **UNILEVER NV**
**Burgemeester s'Jacobplein 1 P.O. Box 760**
**NL-3000 DK Rotterdam(NL)**

(84) **AT BE CH DE ES FR GB GR IT LI NL SE**

Applicant: **UNILEVER PLC**
**Unilever House Blackfriars P.O. Box 68**
**London EC4P 4BQ(GB)**

(84) **GB**

(72) Inventor: **The designation of the inventor has**
**not yet been filed**

(74) Representative: Gambell, Derek et al
**UNILEVER PLC Patents Division P.O. Box 68**
**Unilever House**
**London EC4P 4BQ(GB)**

(54) **Process for the preparation of alkyl glycosides.**

(57) The invention provides a process for preparing an alkyl-glycoside by reacting a monosaccharide with an alkyl alcohol in the presence of a heterogeneous acid catalyst in which monosaccharide and excess alkyl alcohol are heated and reacted in the presence of the heterogeneous catalyst, so that when the reaction mixture is no longer in contact with the catalyst an amount of apolar solvent is added which causes alkyl glycoside to crystallize, separating alkyl glycoside crystals from the mother liquor, removing the apolar solvent from the latter and recirculating the mother liquor from which the apolar solvent has been removed.

Preferably the process monosaccharide and alkyl alcohol are heated and reacted at a temperature between 70 and 120° C.

Preferably the monosaccharide is an aldohexose in particular ,the alkyl alcohol is a C8-C22 alkanol and the heterogeneous catalyst is an acid ion-exchange resin.

EP 0 378 710 A1

**Process for the preparation of alkyl glycosides.**

The invention relates to a process for the preparation of alkyl glycosides by reacting a monosaccharide with an alkyl alcohol in the presence of a heterogeneous acid catalyst.

Such a process is already known from Starch/Stärke 39, (1987) 362-368 which discloses an initial conversion of D-glucose with acetone to a form an isopropylidene derivative and subsequent reaction thereof with a linear alcohol such as 1-octanol in the presence of an ion-exchange resin at 80°C for 16 hours. After filtering off the resin and adding light petroleum the desired octyl glucopyranoside slowly crystallized and was collected. Disadvantage of this process is that as the first step conversion of D-glucose with acetone in a separate step was required, which could only be achieved in a yield of ca 60% and that the acetone liberated during the reaction with octanol can not be easily reused.

In accordance with the present invention alkyl glycosides are prepared by heating and reacting a monosaccharide and excess long chain alcohol in the presence of a heterogeneous acid catalyst, that when the reaction mixture is no longer in contact with the catalyst an amount of apolar solvent is added to the reaction mixture which causes alkyl glycoside to crystallize, followed by separating alkyl glycoside crystals from the mother liquor, removing the apolar solvent from the latter, recirculating this mother liquor and adding further amounts of monosaccharide and alkyl alcohol to the system.

In a preferred embodiment of the invention monosaccharide and alkyl alcohol are heated and reacted at a temperature between 70 and 120°, preferably between 80 and 100°C. The process according to the present invention can be operated batchwise, semicontinuously (cascade metod), but preferably this is done continuously, so that the solid heterogeneous catalyst is reused and that mono- saccharide and alkyl alcohol are added to the recirculated mother liquor before contacting it with the catalyst again.

The alkyl glycoside obtained according to the present invention is of good quality but sometimes the alkyl glycoside crystals obtained are further purified by recrystallization from a suitable solvent such as e.g. water,methanol or octanol.

Suitable starting materials for the present invention are monosaccharides such as pentoses and hexoses, in particular aldohexoses such as glucose, mannose and galactose, also e.g. xylose can be used. Furthermore certain disaccharides can be used especially when under the reaction conditions they are converted to form monosaccharides. Preferably D-glucose is used as the monosaccharide starting material. Suitable higher alkyl alcohols are C8-C22 alcohols, preferably primary, straight chain C12-C18 monohydric alcohols such as 1-octanol, 1-dodecanol, 1-tetradecanol, 1-hexadecanol and 1-octadecanol. Suitable acid heterogeneous catalysts are ion-exchange resins especially sulfonated aromatic resins such as those of the Dowex and Lewatite types. It is desirable before actually using them to make sure that they are dry and in the free $SO_3H$-form by taking appropriate steps.

In order to crystallize the alkyl glycoside from the reaction medium a suitable amount of a apolar solvent is added thereto, such apolar solvent belongs to the group consisting of low boiling hydrocarbons, ethers and lower esters.

The reaction is preferably carried out in a stirred flask equipped with inlets for recirculating ion-exchange resin, mother liquor, monosaccharide and alcohol and an outlet for the reacted mixture leading to filtration unit for the resin and a crystallization tank for the alkyl glycoside which has an inlet for apolar solvent further leading to a filtration unit for the alkyl glycoside crystals and a distillation to remove the apolar solvent from the mother liquor before returning it to the stirred flask optionally via a decolourizing unit.

The reaction route employed in example 1 below is further illustrated by scheme 1 below:

2

Glucose (0.49g)
1-Octanol (0.35g)

1α.H₂O (0.84g)

*Scheme 1. Process scheme (batch) for the preparation of 1α monohydrate. A, reaction; B, filtration; C, crystallisation; D, filtration; E, distillation; F, decolourization; a, equilibrium mixture from the 6 h reaction of 1.8 g D-glucose and 25 mL 1-octanol at 90°C; b, ion exchange resin (Lewatit SPC 108, 0.69 g); c, petroleum ether (200 mL) for washing and crystallisation; d, petroleum ether (20 mL) for washing; e, mother liquor of crystallisation (~ 22 g, mainly 1-octanol).*

## Example 1

10 mmol of anhydrous D-glucose (ex Merck) in 159 mmol 1-octanol (ex Baker) were reacted at 90° C in the presence of 690 mg Lewatite SPC 108 ion-exchange resin (Lewatite is a tradename for a sulfonated aromatic resin from Bayer A.G.), which had previously been washed with 0.5 M aqueous sodium carbonate, 1.0 M aqueous hydrochloric acid subsequently with water and dried to constant weight. After heating and reacting for 6 hours the solid D-glucose particles had disappeared. (After 24 hours complete equilibration had been obtained yielding a mixture containing ca 45% octyl alpha-D-glucopyranoside, ca 19% octyl beta-D-glucopyranoside, ca 2% D-glucose, ca 1% 1,6 anhydro-beta-D-glucopyranose and ca 33% of at least 12 different oligomeric compounds). After removal of the ion-exchange resin to the reaction mixture 8 volumes of light petroleum (b.p. 40-65° C.) were added and octyl alpha-D-glucopyranoside-monohydrate crystallized in a yield of 27% of the theoretical amount. This yield was increased to more than 95% by applying a recirculation system involving distilling off the light petroleum, recirculating the bottom to the reaction vessel and adding equivalent amounts of D-glucose and 1-octanol to account for the octyl glucopyranoside isolated and reacting in the presence of the recirculated ion-exchange resin. This cycle was repeated three times without any decrease in product yield. Thus the first cycle resulted in a 27% yield and the second, third and fourth cycle each gave an almost quantitative yield of the octyl glycopyranoside-monohydrate as white crystals. HPLC showed no accumulation of other compounds and indicated a purity of over 98% Also the mother liquor before recirculating was easily decolourized by treatment with active carbon.

## Example 2

Using the procedure outlined in the previous example 10 mmol of D-glucose (ex Merck), 200 mmol of 1-dodecanol (ex Baker) and 730 mg of a Dowex MSC-1 ion-exchange resin (Dowex is a tradename of Dow Chemical Co and this type is a styrene-divinylbenzene resin with 35% porosity in the SO₃H-form) were reacted for 16 hours at 90° C. The reaction was then stopped by cooling to 30° C, light petroleum was added and the ion-exchange resin removed by filtration and the resin washed with light petroleum which was also added to the filtrate totalling 600 mL. After allowing to cool and rest for 24 hours at 20° C a 52% yield of dodecyl-alpha- and -beta-D-glucopyranoside in a ratio of 2:1 were obtained containing according to

EP 0 378 710 A1

HPLC ca 2% of 1-dodecanol and some oligomeric compounds. After one week a second crop of 8% precipitate was obtained. When instead of light petroleum diethyl ether was used a yield of 29% of mainly dodecyl-alpha-D-glucopyranoside was obtained. Removal of solvent followed by recirculation of the bottom together with the ion-exchange resin and addition of the equivalent quantities of D-glucose and 1-dodecanol to the reactor resulted in almost quantitative conversion into a good quality of dodecyl glucopyranoside according to HPLC as white crystals which were further purified by recrystallization from water.

## Claims

1. A process for preparing an alkyl glycoside by reacting a monosaccharide with an alkyl alcohol in the presence of a heterogeneous acid catalyst characterized in that monosaccharide and excess alkyl alcohol are heated and reacted in the presence of the heterogeneous catalyst, that when the reaction mixture is no longer in contact with the catalyst that an amount of apolar solvent is added which causes alkyl glycoside to crystallize, separating alkyl glycoside crystals from the mother liquor, removing the apolar solvent from the latter and recirculating the mother liquor from which the apolar solvent has been removed.

2. A process according to claim 1 characterized in that monosaccharide, alkyl alcohol are heated and reacted at a temperature between 70 and 120° C.

3. A process according to claim 1 or 2 characterized in that the process is (semi)continuous, that solid heterogeneous catalyst is reused and that monosaccharide and alkyl alcohol are added to the recirculated mother liquor before contacting it with the catalyst again.

4. A process according to claim 1, 2 or 3 characterized in that the alkyl glycoside crystals obtained are recrystallized from a suitable solvent.

5. A process according to any of the claims 1 to 4 characterized in that the monosaccharide is an aldose, preferably an aldohexose.

6. A process according to any of the claims 1 to 5 characterized in that the monosaccharide is D-glucose.

7. A process according to any of the claims 1 to 6 characterized in that the alkyl alcohol is C8-C22 alcohol, preferably a primary, straight chain C12-C18 monohydric alcohol.

8. A process according to any of the claims 1 to 7 characterized in that the heterogeneous acid catalyst is an acid ion-exchange resin.

9. A process according to claim 8 characterized in that the acid ion-exchange resin contains sulfonic acid groups.

10. A process according to any of the claims 1 to 9 characterized in that the apolar solvent comprises a low boiling hydrocarbon.

4

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| A | US-A-3 839 318 (R.C. MANSFIELD) * Column 5, example 3; column 6, example 5; claims 1-6 * | 1 | C 07 H 15/04 |
| A | EP-A-0 132 046 (PROCTER & GAMBLE) * Claims 1-5 * | 1 | |
| A | EP-A-0 239 900 (CPC INTERNATIONAL) * Claims 1-3 * | 1 | |
| A,D | STARCH, vol. 39, no. 10, 1987, pages 362-368, VCH Verlagsgesellschaft mbH, Weinheim, DE; A.J.J. STRAATHOF et al.: "Preparation of long-chain alkyl D-Glucosides by alcoholysis of 1,2:5,6-Di-O-Isopropy-lidene-alpha-D-Glucofuranose" * Page 362, abstract * | 1 | |

TECHNICAL FIELDS SEARCHED (Int. Cl.4)

C 07 H 15/00

The present search report has been drawn up for all claims

| Place of search | Date of completion of the search | Examiner |
|---|---|---|
| THE HAGUE | 29-09-1988 | BRENNAN J. |

EPO FORM 1503 03.82 (P0401)